(19) Europäisches Patentamt / European Patent Office / Office européen des brevets

(11) **EP 4 019 056 A1**

(12) **EUROPEAN PATENT APPLICATION**

(43) Date of publication:
**29.06.2022 Bulletin 2022/26**

(21) Application number: **20383150.8**

(22) Date of filing: **23.12.2020**

(51) International Patent Classification (IPC):
**A61L 26/00** (2006.01)    **A61K 9/06** (2006.01)
**A61K 47/36** (2006.01)

(52) Cooperative Patent Classification (CPC):
**A61L 26/0023; A61K 9/0014; A61K 9/06;**
**A61K 33/38; A61K 47/36; A61L 26/008;**
**A61L 26/0095;** A61L 2400/06; A61L 2400/12

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universitat Politècnica De Catalunya**
**08034 Barcelona (ES)**

(72) Inventors:
• **TZANOV, Tzanko**
**08034 BARCELONA (ES)**
• **IVANOVA, Kristina**
**08034 BARCELONA (ES)**
• **FERRERES CABANES, Guillem**
**08034 BARCELONA (ES)**
• **MORENA GATIUS, Angela Gala**
**08034 BARCELONA (ES)**
• **PEREZ RAFAEL, Silvia**
**08034 BARCELONA (ES)**

(74) Representative: **Ungria López, Javier**
**Avda. Ramón y Cajal, 78**
**28043 Madrid (ES)**

(54) **METHOD TO PRODUCE IN SITU SELF-ASSEMBLED MULTIFUNCTIONAL NANOCOMPOSITE HYDROGEL AND ITS USES THEREOF**

(57)    The present invention provides a method for obtaining *in situ* self-assembled nanocomposite hydrogel with multiple functionalities which comprises nanoparticles (NPs) with phenolic-shell and biopolymers bearing nucleophilic groups. This invention further discloses the hydrogel obtained by the method object of the invention, which comprises 75%-95% by weight biopolymers bearing nucleophilic groups and 5-25% by weight of NPs with phenolic-shell, being the percentages referred to the total weight of the nanocomposite hydrogel. The present invention finally discloses said hydrogel for use as a medicament and, in particular, for the treatment of chronic wounds due to its capability of deleterious enzymes inhibition, antioxidant and antimicrobial properties and fine-tuned mechanical properties.

Fig. 1

**Description**

**OBJECT OF THE INVENTION**

The present invention is directed, in general, to the biomedical field. In particular, the invention relates to a method for *in situ* self-assembling of NPs with phenolic-shell and biopolymers with nucleophilic groups into multifunctional nanocomposite hydrogel for enhancing wound closure and tissue recovery, the multifunctional nanocomposite hydrogel obtained and its uses. In this patent application, the term multifunctional stands for antioxidant activity, antimicrobial activity and inhibition of deleterious enzymes present in chronic wounds.

**TECHNICAL PROBLEM TO BE SOLVED AND BACKGROUND OF THE INVENTION**

Polymer hydrogels are three-dimensional hydrophilic networks prepared by chemical (covalent) or physical (non-covalent) crosslinking of hydrophilic polymers (e.g. polyacrylamide, poly(ethylene oxide), or poly(2-hydroxyethyl methyl acrylate), or biopolymers (e.g. polysaccharides, proteoglycans and/or proteins and its derivatives). Chemically crosslinked hydrogel networks are formed by non-reversible covalent bonds between the polymer chains. Generally, this crosslinking is achieved by redox reactions, radical polymerisation, photo-polymerisation, click chemistry, Michael addition, Schiff's base reactions or enzymes. In spite of showing good mechanical performance for a wide range of applications, the utilisation of toxic catalysts or crosslinkers, and harsh conditions is a major drawback that makes them unsuitable for biomedical applications. In contrast, non-covalent self-assembled hydrogels are formed by intermolecular reversible interactions driven by ionic/electrostatic interactions, polymer chain entanglements, hydrophobic/hydrophilic interactions, hydrogen bonding, host-guest, $\pi$-$\pi$ stacking and metal coordination. The reversible and dynamic interactions in self-assembled hydrogel networks impart properties to these hydrogels including adaptability to environmental changes, self-healing and injectable (shear-thinning) properties. The self-assembling occurs under mild conditions, avoiding the use of harsh chemicals and thereby is suitable for a preparation of materials with wide range of medical applications. Self-assembled hydrogels demonstrate high biocompatibility and can be explored for the *in situ* incorporation of bioactive agents and cells within the matrix. Up to date, most of the self-assembled hydrogels possess poor mechanical properties, slow self-healing properties or require costly and hardly scalable synthesis of the components, which have limited their application in biomedical engineering. Most self-assembling hydrogels are based on natural or synthetic materials establishing host-guest and 'dock-and-lock' interactions.

Chronic wounds such as venous, pressure, arterial and diabetic ulcers require an intensive medical intervention. They present a substantial economic burden to the healthcare systems and in case of untimely and inappropriate treatment, can lead to morbidity and even mortality. All chronic wounds feature prolonged inflammation that lead to accumulation of neutrophils on the site, where they undergo apoptosis and release oxidative enzymes. These enzymes together with reactive oxygen species (ROS) create oxidative stress that contributes to the non-healing state of the wounds. Furthermore, the elevated levels of matrix metalloproteinases (MMPs) and myeloperoxidase (MPO) have a detrimental effect on the wound healing process. In acute wounds, the activity of MMPs is controlled by natural tissue inhibitors (TIMPs), while in the chronic non-healing wounds the ratio MMPs/TIMPs is disturbed leading to uncontrollable degradation of the extracellular matrix and the growth factors. The high MPO concentration, from other side, results in excessive production of hypochlorous acid, which serves to kill bacteria in the first line of defense, but also reacts with most biological molecules, including TIMPs, promoting the proteolytic damage of the healthy tissues. The prolonged exposure of the wound bed to the environment inevitably leads to bacterial colonization and in a worse scenario occurrence of antimicrobial resistant infections.

Therefore, the complexity and the dynamics of the chronic wound environment arises the need for engineering multifunctional approach to efficiently treat the chronic wounds, improving the patient's quality of life and minimizing the healthcare expenditures. The most prospective strategy for chronic wound management should necessarily imply control of oxidative and proteolytic enzymatic activities, keep the wound site free of microorganisms and maintain the site moist, while absorbing wound exudates. Different materials loaded with active compounds including growth factors, for stimulation of cell proliferation and tissue repairing and antimicrobials including antibiotics, silver and metal NPs to control microbial infection have been designed for chronic wound management.

For instance, silver-lignin NPs have been incorporated *in situ* into polyurethane foams to obtain a material for treatment of chronic wounds. The NPs-embedded foams presented sustained silver release over time, strong antibacterial activity, and tuneable swelling capacity as a function of the NPs content. ("A. GALA MORENA, IVAYLO STEFANOV, KRISTINA IVANOVA, SÍLVIA PÉREZ-RAFAEL, MIGUEL SÁNCHEZ-SOTO, AND TZANKO TZANOV. Antibacterial polyurethane foams with incorporated lignin-capped silver nanoparticles for chronic wound treatment. Ind. Eng. Chem. Res. 2020, 59, 10, 4504-4514.Retreived from <https://dx.doi.org/10.1021/acs.iecr.9b06362>.

List of scientific works and patents related to the present invention:

- Scientific document DONGLIN GAN, WENSI XING, LILI JIANG, JU FANG, CANCAN ZHAO, FUZENG REN, LIMING FANG, KEFENG WANG & XIONG LU. Plant-inspired adhesive and tough hydrogel based on Ag-Lignin nanoparticles-triggered dynamic redox catechol chemistry. Nature Communications. 2019. Volume 10, Article number: 1487, retreived from <https://doi.org/10.1038/s41467-019-09351-2>, reports the two-step synthesis of catechol chemistry based hydrogel with long-term adhesiveness to different physiological surfaces, high toughness, and antibacterial activity for enhanced wound healing. The hydrogel is obtained under ambient temperature using acrylic acid, pectin and silver-lignin NPs as precursors. Silver-lignin NPs interact first with ammonium persulphate (APS), producing free radicals that catalyze the polymerization of acrylic acid, and the further gelation of pectin and poly(ethylene glycol) diacrylate. In addition, pectin provides active sites for electrostatic interactions, increasing the flexibility and the toughness of the hydrogel. The method reported in this article requires the use of a chemical initiator and $N_2$ atmosphere for the hydrogel's formation. Furthermore, the use of chemical initiator to create free radicals on the NPs surface is a disadvantage due to the generation of toxic residues.

- Patent application CN110835382A describes a method for preparation of hydrogel dressing for wound management using N-acrylyl glycine that in presence of a photoinitiator (activated by the irradiation with ultraviolet light) polymerizes, forming a network loaded with inorganic NPs such are nano-hydroxyapatite, nano-active glass and nano-clay.

- Patent application WO2011028031A2 provides protocol for formation of a biocompatible and bioadhesive hydrogel with 4-dihydroxyphenyl-L-alanine (DOPA) modified polymers or other phenol derivatives, using an oxidative enzyme (e.g. horseradish peroxidase) for the *in-situ* gelation. The hydrogel demonstrated tissue adhesiveness thanks to modified with/without DOPA derivatives and may find application in the biomedical field.

- Patent application RO134197A2 describes the process for preparation of nanocomposite hydrogels based on collagen and silver NPs for preventing wound infections. The methodology consists in reducing silver ions in the collagen gel with the aid of reducing agents, followed by hydrogen reticulation by a chemical agent. The collagen matrix loaded with antimicrobial silver NPs demonstrated activity against surface adhered and in-suspension Gram-positive and Gram-negative bacteria as well as immunomodulating activity. The hydrogels comprises several drawbacks: i) they require reducing agents such as $NaBH_4$ (which can be toxic) and citric acid, ii) use glutaraldehyde as reticulation agent, iii) silver NPs in the hydrogel are not structural part of the materials and impart only antibacterial functionality.

- Patent application CN104255792A discloses a preparation method for polyphenols modified graphene hydrogel loaded *in situ* with silver NPs for antibacterial applications. The preparation method is simple, cost-efficient and environmentally-friendly, it avoids the use of toxic reducing agents and organic solvents. This hydrogel benefits from the three-dimensional graphene, polyphenols and silver NPs and possess large specific surface area, high conductivity and strong antibacterial activity. The patent claims the modification of graphene with polyphenols, while our hydrogels are based on modified biopolymers. Moreover, the described methodology requires reducing agent, high temperatures (80 - 100 °C) and is time-consuming (4 - 20 h).

- Patent application US10034958B2 describes the synthesis of hemostatic hydrogel compositions containing gelatin or a derivative and silicate NPs useful for treating wounds. The compositions are physically crosslinked gelatin with silicate NPs. In this application, the hydrogels did not contain active NPs, hence, the hydrogels need to include additional pharmaceutical agents in order to provide the desired functionality.

To sum up, the current methods for hydrogel formation possess the following disadvantages:

- Most of the methods reported here are complex, time-consuming, and require the use of chemical reagents as e.g. reducing agents, most of them toxic, to form the NPs.
- The gelation of the hydrogel is triggered either by toxic chemical initiators or enzymes. In the case of enzymatic catalysis, it requires specific reaction conditions to maintain activity that may limit their use.

- Only few examples reported the use of NPs as structural elements in the hydrogel matrix.
- Most of the hydrogels impart antibacterial properties upon the inclusion of antibacterial agent such as silver, in its free form (salt) or in the form of silver NPs. Although being effective antimicrobials, these silver-based materials showed burst release and high toxicity.

In view of the above, there is a need in the art for a new method for obtaining nanocomposite hydrogels using environmentally and human safe hybrid antibacterial and antioxidant NPs playing a dual role as both structural and functional components of the nanocomposite hydrogel.

**BRIEF DESCRIPTION OF THE INVENTION**

The inventors have developed a method that solves the problems of the previously mentioned in the state-of-the-art materials and methods for their production. Thus, the present invention relates to a new method for obtaining an *in situ* self-assembling nanocomposite hydrogel for the treatment of chronic wounds. This method is an *in situ* self-assembling of nanoparticles (NPs) with phenolic shell and biopolymers bearing nucleophilic groups.

In the context of the present invention, the NPs object of the invention that have a phenolic shell are also called phenolic-shell nanoparticles (PSNPs). Thus, in the present invention the PSNPs refers to hybrid metal-phenolic nanoparticles, phenolic nanoparticles and in general other nanoparticles containing phenols groups in their surfaces.

The developed herein *in situ* self-assembling process of PSNPs and biopolymer nucleophilic groups is fast, taking from 0.2 to 180 min and it is performed at mild conditions, avoiding the use of chemical crosslinking, and hazards agents. Therefore, it is suitable for encapsulation of bioactives including drugs such as antibiotics, anti-aging and anti-inflammatory compounds; biomolecules such as growth factors, cytokines, vitamins and proteins, and/or cells for manufacturing materials with application defined activity and improved performance.

In the context of the present invention, a multifunctional nanocomposite hydrogel refers to the hydrogel which is obtained that possess functionalities required for the effective healing of chronic wounds, e.g. high swelling capacity, deleterious enzymes inhibition, antioxidant and antimicrobial properties, and have fine-tuned mechanical properties, allowing for different application scenarios, depending on the formulation used based on the combination of both polymer and PSNPs.

Thus, another object of the invention is the nanocomposite hydrogel for use as a medicament, and particularly, for use in the treatment of chronic wounds. This simple synthetic approach can be easily adopted to other biomedical application and used in the form of ointment, film and coating, among others.

**DETAILED DESCRIPTION OF THE INVENTION**

As explained above, the inventors have developed a method that solves the problems of the previously mentioned in the state-of-the-art materials and methods for their production. This new method, *in situ* self-assembling of nanoparticles (NPs) with phenolic shell and biopolymers bearing nucleophilic groups, is used to obtain a multifunctional nanocomposite hydrogel for the treatment of chronic wounds.

Thus, in a first aspect, the present invention relates to a new method for obtaining an *in situ* self-assembling nanocomposite hydrogel that comprises the following steps:

- Mixing 75-95% by weight of an aqueous solution of biopolymers bearing nucleophilic groups with a concentration of 5 - 20 mg/mL and a pH between 4.5 - 8.0 and 5-25% by weight of an aqueous solution of PSNPs with a concentration of 0.02 - 0.3 mg/mL;
- incubating the mixture obtained in the previous step at 20 - 50 °C for a period of time between 0.2 - 180 min, being the percentages referred to the total weight of the multifunctional nanocomposite hydrogel.

Thus, the present method is a simple, fast and versatile method performed without the presence of any chemical crosslinking of *in situ* self-assembling of polymer-NPs for preparing nanocomposite hydrogels with tuneable physico-mechanical properties.

In a particular embodiment of the present method, the amount of the aqueous solution of biopolymers bearing nucleophilic groups with a concentration of 5 - 20 mg/mL is comprised between 75-95% by weight being the percentages referred to the total weight of the multifunctional nanocomposite hydrogel. In a preferred embodiment, said aqueous solution of biopolymers is comprised between 80%-95% by weight and in a more preferred embodiment, is comprised between 85%-95% by weight.

In a particular embodiment of the present method, the aqueous solution of biopolymers bearing nucleophilic groups has a concentration of 5 to 20 mg/mL, in a preferred embodiment has a concentration of 8.5 to 17.5 mg/mL, and in a more preferred embodiment, has a concentration of 10 to 15 mg/mL.

In a particular embodiment of the present method, the amount of the aqueous solution of PSNPs with a concentration of 0.02-0.3 mg/mL is comprised between 5-25% by weight being the percentages referred to the total weight of the multifunctional nanocomposite hydrogel. In a preferred embodiment, said aqueous solution of PSNPs is comprised between 5-20% by weight and in a more preferred embodiment, is comprised between 5-15% by weight.

In a particular embodiment of the present method, the aqueous solution of the aqueous solution of PSNPs has a concentration of 0.02 to 0.3 mg/mL, In a preferred embodiment, it has a concentration of 0.1 to 0.3 mg/mL, and in a more preferred embodiment, it has a concentration of 0.15 to 0.25 mg/mL

In a particular embodiment of the present method, the pH of the mixture is between 4.0-8.0. In a preferred embodiment the pH of the mixture is between 5.0-7.0, and in a more preferred embodiment, the pH of the mixture is between 5.0-6.0.

In a particular embodiment of the present method, the temperature of the incubation is between 10-50°C. In a preferred embodiment, the temperature of the incubation is between 20-40°C.

In a particular embodiment of the present method, the period of the incubation is between 0.2 to 120 min. In a preferred embodiment, the temperature of the incubation is between 0.2 to 60 min, and in a more preferred embodiment, the temperature of the incubation is between 0.2 to 5 min.

In another aspect, the present invention relates to a method object of this invention that comprises the following steps:

- Providing biopolymers bearing nucleophilic groups;
- Providing phenolic-shell nanoparticles (PSNPs);
- Mixing 75-95% by weight of an aqueous solution of biopolymers bearing nucleophilic groups with a concentration of 5 - 20 mg/mL and a pH between 4.5 - 8.0 and 5-25% by weight of an aqueous solution of PSNPs with a concentration of 0.02 - 0.3 mg/mL;
- incubating the mixture obtained in the previous step at 20 - 50 °C for a period of time between 0.2 - 180 min, being the percentages referred to the total weight of the multifunctional nanocomposite hydrogel.

In case the biopolymers used in the method object of the invention which do not bear nucleophilic groups, said biopolymers should be prepared based on the method described in the article SEI KWANG HAHN, JUNG KYU PARK, TAKASHI TOMIMATSU, TSUYOSHI SHIMOBOJI. Synthesis and degradation test of hyaluronic acid hydrogels. International Journal of Biological Macromolecules. March 2007. Volume 40, Issue 4, 10, Pages 374-380. Retrieved from <https://doi.org/10.1016/j.ijbiomac.2006.09.019>, at a room temperature, during 2 h, obtaining the biopolymers bearing nucleophilic groups.

The nanoparticles that are used in the present method are characterised by a phenolic shell that interacts with the nucleophile groups from the biopolymers.

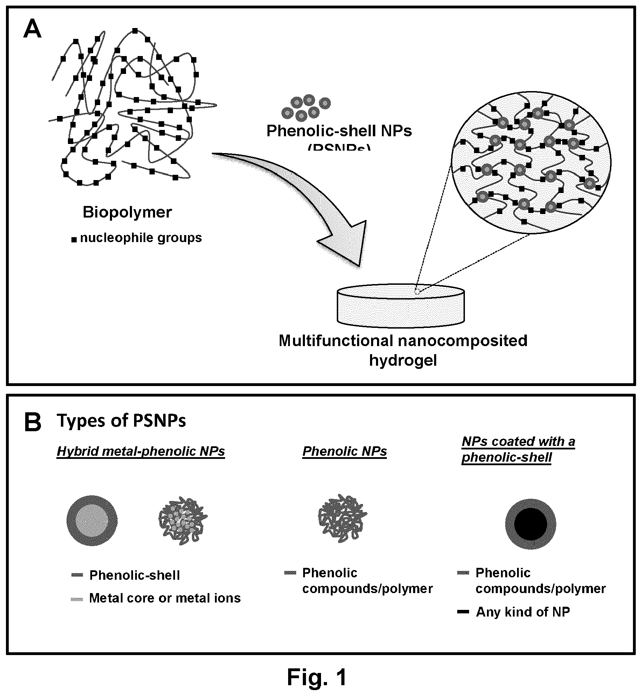

Thus, in particular embodiments of the present invention, these PSNPs that comprises a phenolic shell are nanoparticles made of phenolic compounds, hybrid metal-phenolic nanoparticles or another nanoparticles containing phenolic groups in their surface (Figure 1). The nanoparticles that comprises a phenolic shell and a metal core, which in the context in the present invention is called hybrid metal-phenolic nanoparticles (MPNPs), are prepared based on the method described in the articles: HOYO J, IVANOVA K, TORRENT-BURGUES J, TZANOV T. Interaction of Silver-Lignin Nanoparticles With Mammalian Mimetic Membranes Front Bioeng Biotechnol. 2020; 8: 439. Retreived from <https://doi.org/10.3389/fbioe.2020.00439> and LI, KE, ET AL. Self-Assembled Metal-Phenolic Nanoparticles for Enhanced Synergistic Combination Therapy against Colon Cancer. Advanced Biosystems. November 2018; 3(2): 1800241. Retrieved from <https://doi.org/10.1002/adbi.201800241>. In the context of the present invention, these metal-phenolic nanoparticles are synthetized at a temperature between 25-40 °C for 2-72 h under continuous agitation or enhanced by ultrasound using natural phenolic compounds as reducing agents or ligands and capping agents instead of less environmentally friendly chemical reagents.

In other particular embodiments of the invention, the method uses nanoparticles (NPs) that comprises a phenollic shell and a phenollic core, which in the context of the present invention are called phenolic nanoparticles (NPs) or phenolic NP. These phenolic nanoparticles can be synthesized under continuous agitation, sonication or by other methods as solvent evaporation etc. In particular embodiments of the present invention, they are synthesized from phenolic compounds using enzymatic or sonication process for 1h at 50-55°C. In the context of the present invention, functional PSNPs refers to the antioxidant and antibacterial properties of the nanoparticles that are used in the present method.

In particular embodiments, the phenolic-shell nanoparticles can be obtained by coating any kind of nanoparticle with an external layer of phenolic compounds able to interact with the nucleophile groups from the biopolymer.

This method object of the invention can be performed in a recipient with a volume proportional to the amount of hydrogel that it is going to be prepared. On the other hand, this method object of the invention can also be performed directly on the surface of a scaffold, medical devices...etc., to which an antibacterial or healing function is to be conferred. In a preferred embodiment, this method object of the invention can be performed directly on the surface of a scaffold and medical devices.

The present invention also refers to a nanocomposite hydrogel which is characterized in that it comprises 95-75% by weight of biopolymer bearing nucleophilic groups and 5-25% by weight of hybrid PSNPs, being the percentages referred to the total weight of the of the multifunctional nanocomposite hydrogel. Thus, another aspect of the present invention is also refers to a nanocomposite hydrogel obtained from the method disclosed in the present document, which is characterized in that it comprises 95-75% by weight of biopolymer bearing nucleophilic groups and 5-25% by weight of hybrid PSNPs, being the percentages referred to the total weight of the of the multifunctional nanocomposite hydrogel.

The PSNPs of the hydrogel are both structural and functional components.

In particular embodiments, the hydrogel can be obtained as an ointment or freestanding films. In another preferred embodiment, the nanocomposite hydrogel can be obtained as coating which covers the surface of different kind of scaffolds, depending on the intended use. In this last embodiment, the scaffolds to be coated are embedded in a recipient with the reagents that are used in the method object of the invention and are incubated in the conditions disclosed above.

The nanocomposite hydrogel can be also deposited on the surface of catheters, implants, etc. using different coating techniques such as layer-by layer, sonochemistry, spray coating, dip- and spin-coating, spread coating among others. In addition, due to their injectability properties, the hydrogel can be used to prepare materials and modify or cover different kind surfaces with bioprinting and electrospinning techniques.

Different biopolymers can be used to form the hydrogel composition. In particular embodiments, the biopolymer could be a modified or natural biopolymer, wherein said biopolymer bears nucleophilic groups or is modified with nucleophilic groups. In a particular embodiment, the biopolymer bearing nucleophilic groups is selected from the group consisting of a polypeptide, a polysaccharide, a derivative thereof and combinations thereof. In a preferred embodiment, wherein the biopolymer is a polypeptide, it is selected from the group consisting of collagen, actin and fibrin. In another preferred embodiment, wherein the biopolymer used for forming the hydrogel is a polysaccharide, said biopolymer is selected from the group consisting of hyaluronic acid, chondroitin, cellulose, alginate and chitosan.

The self-assembling event would be possible with any nucleophile group such as alcohols, phenols, carboxylic, amines or thiols bearing modified or natural biopolymer. In a preferred embodiment, a derivative namely hyaluronic acid (HA-SH) modified with thiol groups, was employed to form the hydrogel composition. Nevertheless, the biopolymers that are used for obtaining the hydrogel composition are not limited to the thiolated polymers.

In particular embodiments, the phenolic shell of the PSNPs is made of compounds with phenolic groups, which in the present document refers to natural phenolic polymers, such as lignin or phenolic compounds, such as gallic acid (GA), epigallocatechin gallate (EGCG), tannic acid, ellagic acid among others.

The PSNPs used in the method of the present invention may comprises a metal core.

In a particular embodiment, the method object of the invention uses hybrid nanoparticles (NPs) comprising a metal core and a shell of phenolic compounds, preferred a shell of natural phenolic compounds, which serve as both structural and active component in the hydrogel nanocomposite. In the context of the present invention, these nanoparticles with metal core and a shell of phenolic compounds are called hybrid metal-phenolic nanoparticles (MPNPs).

In one embodiment of the present invention, non-limiting examples of metals suitable for the metal core are Ag, Cu, Co, Zn, Fe, Se, Te, Al, Au, V, Cr, Mn, Ni, Zr, Mo, Ru, Rh, Cd, Ce, Eu, Gd, Tb among others. In a particular embodiment, the metal core of the MPNP is made of a metal that is selected from the group consisting of Ag, Cu, Co, Zn, Fe, Se, Te, Al, Au, V, Cr, Mn, Ni, Zr, Mo, Ru, Rh, Cd, Ce, Eu, Gd, and Tb. In preferred embodiments, the metal core is made of a metal that is selected from the group consisting of Ag, Cu, Co, Zn, Se and Te. In preferred embodiments, the metal core is made of a metal that is selected from the group consisting of Ag, Cu and Co, and more preferred embodiments, the metal core is made of Ag.

The metal core shows different activities in biomedical applications, such as a strong antibacterial activity, such as Ag, Cu, Co, Zn, Se and Te, diagnostic such as Au, Fe or even theranostic, which makes the composition object of the invention, very suitable for use in the effective treatment of chronic wounds.

In particular embodiments, the phenolic shell of the PSNPs with a metal core can be made of compounds with phenolic groups which are able to form nanoparticles (NPs) with different metals, which in the present document refers to natural phenolic polymers, such as lignin, or phenolic compounds, such as gallic acid (GA), epigallocatechin gallate (EGCG), tannic acid, ellagic acid among others which are able to reduce the metal ions and/or form metal phenolic networks.

In another particular embodiment of the present invention, the PSNPs that are made of phenolic compounds with accessible phenolic groups in their shell, are the called phenolic nanoparticles or phenolic NPs. In this particular embodiment, the phenolic NPs are made of natural phenolic compounds, which serve as both structural and active component in the nanocomposite hydrogel.

In particular embodiments, the phenolic NPs and the phenolic shell of the nanoparticles used in the present invention can be made of phenolic compounds such as gallic acid, rutin, epigallocatechin gallate, tannic acid, ellagic acid, lignin and its derivarites among others. In preferred embodiments, the phenolic NPs and the phenolic shell of the NPs are made of phenolic compounds that are selected from a group consisting of gallic acid, rutin, epigallocatechin gallate, tannic acid, ellagic acid, lignin and its derivarites. In more preferred embodiments, the phenolic NPs and the phenolic shell of the NPs are made of phenolic compounds that are selected from the group consisting of gallic acid, rutin, epigallocatechin gallate, tannic acid, ellagic acid and lignin.

These phenolic-shell nanoparticles with phenolic core are produced using a technologically simple and environmentally friendly safe-by-design approach.

In other embodiments, the phenolic shell of the MPNPs that is made of natural phenolic compounds confers antioxidant activity, as well as enhanced stability and biocompatibility in comparison to the stand-alone metallic NPs.

In particular embodiments, these MPNPs are produced using a technologically simple and environmentally friendly safe-by-design approach for simultaneous synthesis and capping the metal core of the NPs with natural phenolic compounds to yield highly concentrated and biocompatible hybrid MPNPs with enhanced stability and antimicrobial activities compared to pristine metal-NPs. The phenolic shell of the MPNPs imparts colloidal stability to the antimicrobial metal in the NPs core, provides antioxidant properties and improves the biocompatibility of hybrid MPNPs compared to the metal NPs alone. The present invention goes beyond the reported up to date self-assembling polymer platforms, since the obtained MPNPs are from one side the driving force for the *in-situ* self-assembling with nucleophilic groups-bearing biopolymers and consequent gelation, and from another side an active agent, providing antibacterial/antioxidant properties to the hydrogel nanocomposite.

The technical feature that should be highlighted in the present invention is that due to the phenolic shell of the PSNPs the hydrogel is formed. The phenolic shell of the NPs acts as a driving force for the *in situ* self-assembling of the MPNPs with the nucleophilic groups of biopolymers and, consequently, said phenolic shell is the responsible of the self-assembling hydrogel composition. Thus, the developed herein *in situ* self-assembling approach based on PSNPs and biopolymer nucleophiles avoids the use of chemical crosslinking, that are hazardous agents, and limit the use of the hydrogel in human's body.

In contrast, the hydrogel of the present invention, due to the lack of chemical crosslinking agents, as they are not necessary for the formation of the hydrogel, it can be used on human body for the effective healing of chronic wounds.

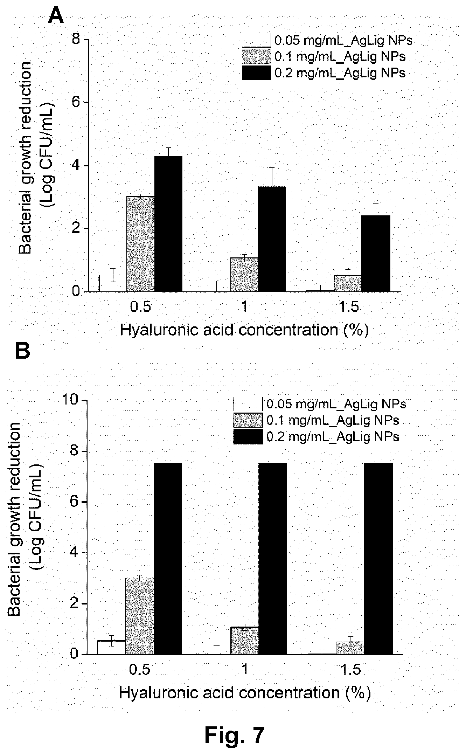

In the context of the present invention, chronic wounds refer to venous, pressure, arterial and diabetic ulcers that require an intensive medical intervention. In this regard, the hydrogel composition presents a high swelling capacity, deleterious enzymes inhibition, antioxidant and antimicrobial properties, and have fine-tuned mechanical properties as it is disclosed in figures 7-12, allowing its use for different biomedical applications related with healing, depending on the formulation used based on the combination of both polymer and NPs.

Thus, the simple, rapid and versatile synthetic route for the hydrogel formation in combination with their biocompatible and tailored functionality in accordance to the compounds employed in the *in situ* self-assembling opens the possibility for their application in variety of medical fields.

Thus, another aspect of the present invention is the hydrogel of the invention for use as a medicament. The present invention also relates to the use of the hydrogel of the invention in the manufacture of a medicament. Alternatively, this aspect may be reformulated as a method of treatment chronic wounds which comprises administering the hydrogel of the invention.

Therefore, the hydrogel composition has multiple functionalities such as antioxidant activity, antimicrobial activity towards clinically relevant bacterial strains and inhibition of deleterious enzymes.

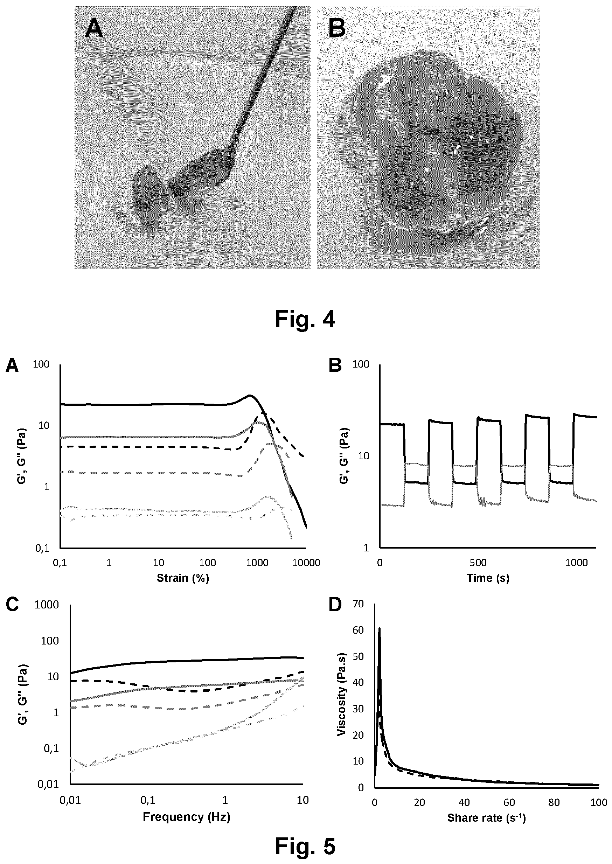

Based on what it has been previously disclosed, in preferred embodiments, when the hydrogel composition is obtained as an ointment, the hydrogel can be applied to any kind of surface that needs to be treated. In the contexts of the present invention, the "any kind of surface" refers to skin, injury, chronic wound, the surface of medical devices, dressings (bandages), among others. In another embodiment, when the hydrogel can be obtained as an injectable gel and employed for bioprinting and electrospinning. In other preferred embodiment of the present invention, the hydrogel composition is obtained as a coating on different surfaces, being in the present context, implants, dressings (bandages), facial masks, among others. These embodiments are demonstrated in figures 4 and 5 of the present document. In a more preferred embodiment, the hydrogel is presented as a wound dressing.

In other preferred embodiments, the hydrogel composition is also suitable for the encapsulation of human cells or other biomolecules, such us growth factors, cytokines, vitamins, proteins among others.

These hydrogels present the following properties and advantages over the state of art:

- The PSNPs act as an initiator of the hydrogel gelation avoiding the use of external gelators, usually hazard and toxic reagents.
- The NPs are not only structural but also active agent in the hydrogel, imparting antimicrobial and antioxidant activity.
- The hydrogel object of invention has the capability to inhibit deleterious enzymes involved in the wound chronicity such as myeloperoxidase and matrix metalloproteases. All these properties are beneficial for the treatment of chronic wounds.
- Contrarily to the most hydrogels for wound management, the active agent of the invention is in a nano-form. The nano-form presents advantages towards the bulk form of the compound, due to the enhanced reactivity associated with the high surface to volume ratio. In these nanocomposite hydrogels, the active agent is also a structural element, thus allowing for controlled release, reducing potential toxicity of the metal.
- The use of the biopolymers and natural compounds, present either in the NPs shell or in the hydrogel matrix, improves the biocompatibility of the final product.
- This hydrogel can be easily synthesized *in situ* using a wide range of polymers, metals and phenolic compounds.
- The hydrogels can be prepared in different forms (wound dressing, topical ointment, coating for medical devices and injectable hydrogels), by following the recipe and preparation method stated in this document. The hydrogels coatings can be formed onto flat or curved surfaces, including catheters, etc. The coatings provide functional properties to inert surfaces.
- The physico-mechanical properties of the hydrogels can be tuned by simply adjusting the formulation.
- The hydrogels can be used alone or as active ingredients in other biomedical formulation, e.g. creams and lotions for cosmetic products.

## DESCRIPTION OF THE FIGURES

To complete the description and for the purpose of facilitating a better understanding of the features of the invention, this specification is accompanied by a set of figures, as an integral part thereof, where by way of non-limiting example, the following has been represented:

**Figure 1.** A) Schematic representation of self-assembling interaction between biopolymer and phenolic-shell NPs (PSNPs) driving to the hydrogel formation. B) Types of PSNPs able to interact with nucleophile groups from biopolymers to form nanocomposite hydrogels.

**Figure 2.** Example of the gelation process of HA and its derivatives upon mixing with AgLig NPs. A) Control: mixing pristine HA with AgLig NPs did not resulted in gelation. B) Self-assembling event: Mixing of HA-SH with AgLig NPs resulted in the hydrogel formation.

**Figure 3.** Example of the gelation using thiolated hyaluronic acid and A) EGCGCo NPs and B) PheLigNPs.

**Figure 4.** Shear-thinning (A) and self-healing properties after injection through the needle (B) of the HA-SH/AgLig NPs hydrogels.

**Figure 5.** Rheological tests of HA-SH/AgLig NPs hydrogels. A) Strain sweep measurements showing the strong strain overshoot behaviour with both G' and G" local maximum; B) Cyclic strain time sweep measurements, performed with 0.2 and 2000 % alternating strains; C) Frequency sweep measurement revealed rise of G' and G" with increasing the frequency and D) viscosity vs shear rate rheological test of hydrogels, prepared with 1.5 % HA-SH and two different concentrations of NPs at 25 or 37 °C.

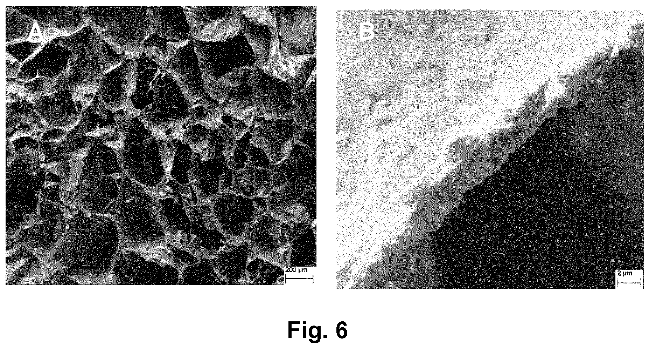

**Figure 6.** SEM images of hydrogels prepared with 1.5% of HA-SH and 0.2 mg/mL_AgLig NPs. A) hydrogel cross-section and B) zoom of the pore's wall cross-section.

**Figure 7.** Antibacterial activity of the hydrogels against A) *S. aureus* and B) *P. aeruginosa* of hydrogels with three different concentrations of HA-SH (1.5, 1.0 and 0.5 %) and AgLig NPs (0.05 -0.2 mg/mL).

**Figure 8.** Antibacterial activity of the hydrogels against *S. aureus* (dark grey) and P. *aeruginosa* (light grey). 1.5 % concentration of thiolated hyaluronic acid and 0.2 mg/mL of gallic acid-cobalt NPs (GACo), epigallocatechin gallate-cobalt NPs (EGCGCo) and epigallocatechin gallate-copper NPs (EGCGCu).

**Figure 9**. DPPH inhibition (%) after 1, 2 and 24 h incubation with 1.5 % HA-SH hydrogels, prepared with 0.05 and 0.1 mg/mL_AgLig NPs.

**Figure 10.** *Ex-vivo* determination of MMP and MPO residual activity in wound fluid after 1h and 24 h incubation with 30 mg of HA-SH/AgLig NPs hydrogels with three different concentrations of HA-SH (1.5, 1.0 and 0.5 %) and AgLigNPs (0.2, 0.1 and 0.05 mg/ml).

**Figure 11.** Inhibition of deleterious enzymes of different HA-SH/PSNPs hydrogels with 1.5% of HA-SH and 0.2 mg/ml of PSNPs: A) MPO and B) MMPs. Gallic acid-cobalt NPs (GACo), epigallocatechin gallate-cobalt NPs (EGCGCo) and epigallocatechin gallate-copper NPs (EGCGCu).

**Figure 12.** Cumulative release of silver from the hydrogels. Hydrogels with different concentrations of HA-SH (1.5, 1 or 0.5 %) and A) 0.2 mg/mL_AgLig NPs, B) 0.1 mg/mL_AgLig NPs or C) 0.05 mg/mL_AgLig NPs incubated for up to 5 days in PBS (grey lines) or PBS hyaluronidase (black lines).

**Figure 13.** Viability of keratinocytes after 1, 3 and 7 days of direct contact with HA-SH/ AgLig NPs hydrogels assessed by (A) AlamarBlue and (B) Live/Dead kit assays. The green and red fluorescence images are overlaid. Due to the black/white transformation of the image, the light grey colour corresponds to live cells and dark grey to dead cells. Scale bar corresponds to 100 μm.

**Figure 14.** Viability of keratinocytes after 1, 3 and 7 days of direct contact with different HA-SH/ PSNPs hydrogels assessed by AlamarBlue. Gallic acid-cobalt NPs (GACo), epigallocatechin gallate-cobalt NPs (EGCGCo) and epigallocatechin gallate-copper NPs (EGCGCu).

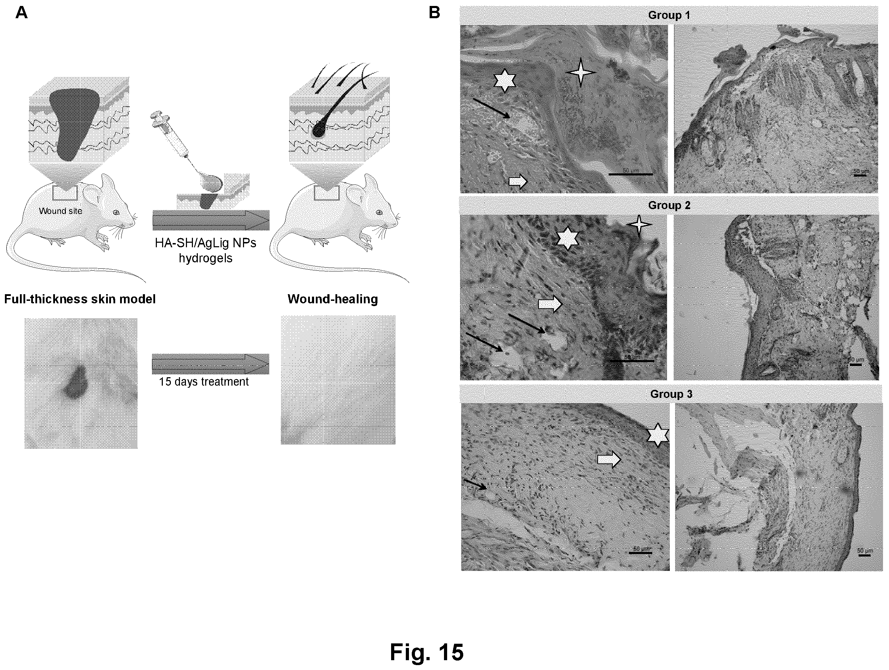

**Figure 15.** A) Scheme on the *in vivo* mouse model (top) and representative photos of full thickness skin wounds before and after application of the nanocomposite hydrogels. The images taken after 15 days of treatment indicate hair follicle regeneration, sebaceous gland development and non-sign of inflammation. B) Hematoxylin-eosin-stained sections of the wound tissues at sacrifice (day 15th post-surgery). Histology of excised wound tissue taken from: non-diabetic mouse treated 3 times per day with Dermazin (group 1), non-diabetic mouse treated 3 times per day with nanocomposite hydrogel (group 2) and diabetic mouse treated 3 times per day with nanocomposite hydrogel (group 3). Grey 4 - point stars - indicate the scab, grey 6 - point stars indicate the epidermis, grey arrows indicate new granulation tissue and the black arrows indicate the blood vessels. The images were taken at different magnifications and the scale bar corresponds to 50 μm.

## DESCRIPTION OF AN EXEMPLARY EMBODIMENT OF THE INVENTION

For the purpose of helping to better understand the invention, and according to a practical embodiment thereof, exemplary preferred embodiments are enclosed as an integral part of description, being illustrative and never limiting of the invention.

**Experiment 1: Synthesis of thiolated hyaluronic acid**

At first, adipic acid dihydrazide functionalized hyaluronic acid (HA-ADH) was prepared as described elsewhere. Briefly, hyaluronic acid (HA) was dissolved in MilliQ water to obtain 5 mg/mL HA solution. An appropriate amount of adipic acid dihydrazide (ADH) (45-fold molar excess) was added to the solution and left under stirring for 30 min until dissolved completely. The pH of the mixture was adjusted to 4.8 by addition of 1M hydrochloric acid (HCl). After that, four times molar excess of N-(3-dimethylaminopropyl)-N'-ethylcarbodiimide was added in solid form and the pH of the reaction mixture was maintained at 4.8 by adding 1M HCl. The reaction was stopped after 2 h by raising the pH to 7.0 with 1M of NaOH. HA-ADH was recovered by acetone/NaCl precipitation from the solution. Then, the HA-ADH solution was transferred to pre-washed dialysis membrane (cut off 13 kDa) and dialyzed against distilled water for one day. The resulting solution was finally lyophilized for 3 days and stored at 4 °C under nitrogen atmosphere until further use.

The thiolation of HA-ADH to obtain thiolated HA (HA-SH) was carried out in a one-step coupling reaction between Traut's reagent and the primary amino groups of ADH. Briefly, 5 mg/mL HA-ADH solution was prepared in MilliQ water. Then, 2.5 mL of phosphate buffer (PB, 100 mM, pH 8) containing Traut's reagent was added to the HA-ADH solution. The molar ratio of -ADH to Traut's reagent was 1:2. After 2 h, HA-SH product was purified by dialysis against 100 mM NaCl pH 5.5 for one day. Finally, the product was lyophilized and stored at 4 °C under nitrogen. Finally, the incorporation of free thiol groups in the polymer chain was confirmed by Ellman's reagent.

**Experiment 2: Synthesis of silver-lignin nanoparticles (AgLig NPs)**

The synthesis of AgLig NPs was carried out for 3 days at 60 °C under stirring. Lignin solution (10 mg/mL) was prepared in MilliQ water and the pH was adjusted to 5.5 with 5 M HCl. Then, 20 mL of 2 mg/mL aqueous $AgNO_3$ solution in MilliQ were added to 30 mL of lignin solution and the reaction was performed under continuous stirring. Finally, AgLig NPs were recovered by centrifugation at 18000 rpm for 45 min. The pellet was resuspended in MilliQ water and re-dispersed in a ultrasound bath for 10 min. The NPs concentration (mg/mL) was determined measuring the weight of the AgLig NPs pellet obtained after lyophilisation of AgLig NPs solution.

The obtained AgLig NPs have a Z potential value above -40 mV and an average size of 20 nm determined by TEM analysis. Furthermore, the phenol content was quantified using Folin-Ciocalteu method and the XPS experiments confirmed the presence of metallic silver.

**Experiment 3: Synthesis of EGCGCo, EGCGCu and GACo NPs**

The hybrid metal-phenolic nanoparticles were synthesised with different metal ions and phenolic compounds. The metallic salt (cobalt chloride (Co) or copper chloride (Cu)) and the phenolic compound (gallic acid (GA) or Epigallocathechin-gallate (EGCG)) were dissolved in ethanol (20mM) and mixed in a ratio of 2:3 (metallic salt: phenolic compounds), the mixtures were stirred until they were completely dissolved. After that the pH was raised to 7 using NaOH and reacted at 37 °C for 24 hours. After that, the particles were centrifuged and wash with water twice and resuspended in miliQ water.

The hybrid metal-phenolic nanoparticles obtained presented negative Z potential (above -30 mV) and average size of 40 nm. Finally, the phenol content was estimated with Folin-Ciocalteu method and the metal content with ICP-MS.

**Experiment 4: Synthesis of PheLigNPs**

Phenolated lignin nanoparticles (PheLigNPs) are prepared in a two-step sonochemical method based on laccase/mediator system. Acetosyringone, which is a small phenolic compound used as mediator, is first dissolved at 1.5 g/L in 50 mM, pH 5 sodium acetate buffer. Once the mediator is dissolved, lignin (10 g/L) is added to the mixture. The enzyme laccase is then added and the mixture is incubated at 50 - 55 °C for 1 h to pre-activate lignin. Tannic acid, a high molecular weight phenolic compound, is added at a 1:1 tannic acid to lignin ratio. The solution is then subjected to high-intgensity ultrasounds for 1 h at 50 - 55 °C. The phenolated lignin nanoparticles are purified by centrifuging the samples at 20000 xg for 15 min. After resuspending the resultant pellet in water, the samples were subjected to low speed centrifugation to discard the largest particles.

The PheLigNPs present a diameter ranging from 150-200 nm. The phenolic content estimated by Folin-Ciocalteu method of the nanoparticles showed a 162 % increase on phenolic content in comparison with non-treated lignin.

The nanoparticles inhibit the growth of Gram-positive *S. aureus* and Gram-negative P. *aeruginosa* and *E. coli*, presenting a minimum inhibitory concentration of 1 mg/ mL.

## Experiment 5: Preparation of hydrogels

The hydrogels were prepared by simply mixing HA-SH and NPs solutions. Briefly, 0.9 mL of HA-SH (5 - 15 mg/mL) in 0.1 M acetate buffer, pH 5.5 is mixed with 0.1 ml of PSNPs (0.02 - 0.2 mg/mL) and incubated at 37 °C for 2 h. The gels formation was monitored every 10 min.

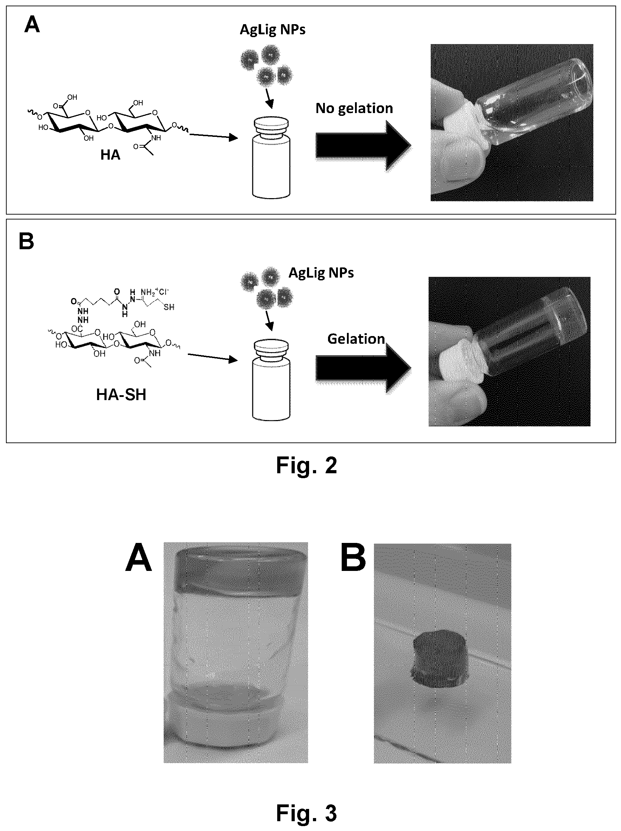

Using this methodology different hydrogels combining HA-SH with different molecular weight (from 40 to 700 kDa) and PSNPs were prepared. Figure 2 and 3 show the formation of nanocomposite hydrogels made of HA-SH with hybrid metal-phenolic nanoparticles as AgLig NPs and EGCGCo and with the PheLigNPs made of phenolic compounds.

## Experiment 6: Rheological characterization of HA-SH/AgLig NPs hydrogels

Rheological characterization of the hydrogels was performed with an ARG2 rheometer (TA instruments, UK), equipped with electrical heated plates. The samples were analyzed in parallel plate geometry (40 mm). The viscoelastic behavior of the hydrogels was explored by setting the rheometer at constant frequency (1 Hz) and in a range of increasing strains (0.1-5000 %), at monitoring the change of G' and G". Structural changes of the hydrogels were considered when deviation from the linear behavior was observed. With the aim of exploring their self-healing properties, the hydrogels were subjected to a cyclic strain time sweep measurements with an alternating strain (0.2 - 2000 %). The upper strain value used to perform the cyclic strain sweep measurements was chosen, according to the obtained strain sweep test results (a value which is out of the linear viscoelastic region). Frequency sweep measurements were performed at constant strain (1 %) in a range of 0.01 - 10 Hz. Continuous flow of the hydrogels was examined by monitoring the change of hydrogels viscosity as a function of the increasing shear rate (0.01 - 50 $s^{-1}$). All experiments were performed at 37 °C, while the continuous flow behavior was additionally studied at 25 °C. The analysis of the obtained data was conducted using TA Instruments TRIOS software. The rheological results obtained in the study of HA-SH/AgLig NPs hydrogels (Figure 5) confirmed the self-healing and shear-thinning properties observed during the manipulation of this material (Figure 4) and tuneable physico-mechanical properties depending on polymner/NPs composition.

## Experiment 7: Antibacterial activity

The potential of the hydrogels to inhibit bacterial growth was assessed by a standard flask shake method (ASTM-E2149-01) with some modifications. Single colonies of S. *aureus* and *P. aeruginosa* were inoculated overnight in 5 mL sterile Mueller Hinton Broth and incubated at 37 °C with shaking (110 rpm). Then, the bacteria were diluted in sterile PBS until absorbance of $0.005 \pm 0.01$ at 600 nm was reached, which corresponds to 6 x $10^5$ colony-forming unit (CFU) per mL. Thereafter, the hydrogels were incubated with 0.2 mL of bacterial suspension at 37 °C and 230 rpm for 1 h. The determination of the inoculum cell density of the suspensions was carried out by withdrawing part of the suspension before introducing the hydrogels and after 24 h in contact with them. The withdrawn suspensions were serially diluted in sterile PBS solution, plated on a Baird-Parker agar or Cetrimide agar and incubated at 37 °C for 24 h to determine the number of viable bacteria. The antibacterial activity is reported in terms of bacterial reduction (Log CFU/mL) calculated as the difference between the number of bacteria before and after the incubation with the samples.

The antimicrobial activity of the HA-SH/AgLig NPs hydrogels was assessed against two clinically relevant bacterial strains, frequently identified in chronic wounds - the Gram-positive *S. aureus* and Gram-negative P. *aeruginosa.* The hydrogels prepared with 0.2 mg/mL AgLig NPs displayed the strongest bactericidal activity, reducing *P. aeruginosa* and *S. aureus* bacterial growth by 7 and 4.5 log, respectively. (Figure 7) In the case of the thiolated hyaluronic acid hydrogels synthesized using copper and cobalt PSNPs, the antimicrobial activity was lower than the silver containing NPs, however, the hydrogels with 0.2 mg/ml of particles were able to inhibit the growth of both Gram-positive *S. aureus* and Gram-negative *P. aeruginosa.* All the hydrogels prepared by the method disclosed in this patent show antimicrobial activity due to the incorporation of PSNPs.

## Experiment 8: Antioxidant activity

The radical scavenging activity of the hydrogels and the starting materials was determined spectrophotometrically measuring the decrease of the absorbance of the 1,1-diphenyl-2-picrylhydrazyl (DPPH) radical at 517 nm. Briefly, 30 mg of each hydrogel were incubated in 60 $\mu$M DPPH solution in methanol at room temperature in dark conditions for 30 min. All measurements were performed in triplicate. The following equation was used to calculate the antioxidant activity:

$$\text{DPPH inhibition (\%)} = [1 - (A/Ao)] \times 100$$

where Ao is the absorbance of the negative control (DPPH solution alone) and A is absorbance of DPPH, incubated with the hydrogels.

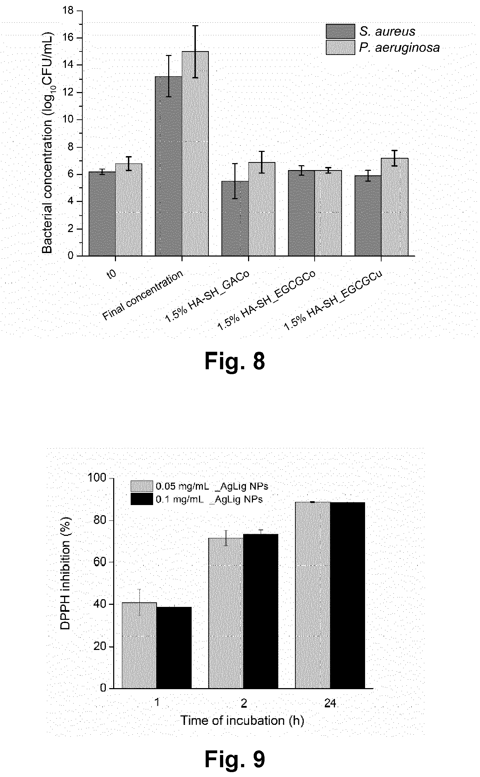

The results indicated that the nanocomposite hydrogels HA-SH/AgLig NPs showed radical scavenging activity due to the inclusion of the antioxidant AgLig NPs in its matrix (Figure 9).

**Experiment 9: Collagenase and myeloperoxidase inhibition**

The inhibitory efficiency of the hydrogels against collagenase and MPO was assessed in wound exudates extracted from the dressing (UrgoClean from Urgo Medical) of a patient with a venous leg ulcer, provided by Hospital de Terrassa (Spain). After removing the dressing from the patient's limb, 1 g of specimen containing the exudate was soaked in 5 ml MilliQ water for 10 min. Thereafter, the mixture was vortexed for 3 min and centrifuged for 5 min at 10 000 rpm. The extracted liquid was collected and centrifuged once again for 5 min at 10 000 rpm to remove the sludge from the wound dressing and stored at 4 °C for further use.

The inhibitory efficiency of the hydrogels against collagenase was estimated by measuring the increase in the fluorescence intensity of fluorescently labeled gelatin (EnzChek® substrate) cleaved in presence of active collagenase. Briefly, 30 mg hydrogel samples were incubated for 24 h at 37 °C in 400 $\mu$L collagenase (49 U/mL) dissolved in 10 mM Tris-HCl buffer, pH 7.8. Afterwards, 100 $\mu$L of the solution were transferred to 96-well microplate, diluted with 80 $\mu$L 10 mM Tris-HCl buffer, pH 7.8, and mixed with 20 $\mu$L of 250 (g/ml gelatin substrate solution. The fluorescence was read at $\lambda$excitation/emission = 493/528 nm using microplate reader Infinite M200 (Tecan, Austria). All measurements were performed in triplicate and the results were expressed as collagenase residual activity compared to the control collagenase solution without hydrogel.

The *ex-vivo* inhibitory activity of the hydrogels against MPO was determined by quantifying the amount of taurine chloramine, produced by the MPO/H2O2/Cl- system. Briefly, 270 $\mu$L of 50 mM PBS pH 6.5 with 150 mM NaCl and 5 mM taurine were mixed with 30 $\mu$L 1 mM H2O2 and 4 $\mu$L MPO solution, in presence of 30 mg hydrogel samples. The samples were incubated for 30 min at 37 °C and the enzymatic reaction was stopped by addition of 10 $\mu$L 1 mg/mL catalase solution. Then, 75 $\mu$L of the detection reagent (2 mM 3,3',5,5'-tetramethylbenzidine in 10 % DMSO, 100 (L NaI in 400 mM acetate buffer pH 5.4) were added to the reaction mixture. After 5 min the amount of taurine chloramine generated during the enzymatic reaction was measured by recording the absorbance at 650 nm. All measurements were carried out in triplicate and the results were expressed as a percentage of MPO inhibition, compared to controls without hydrogels, where the activity was considered as 100 %.

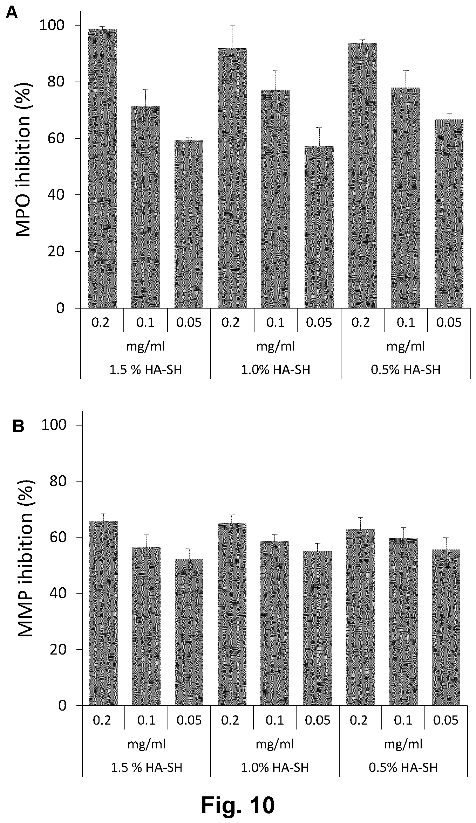

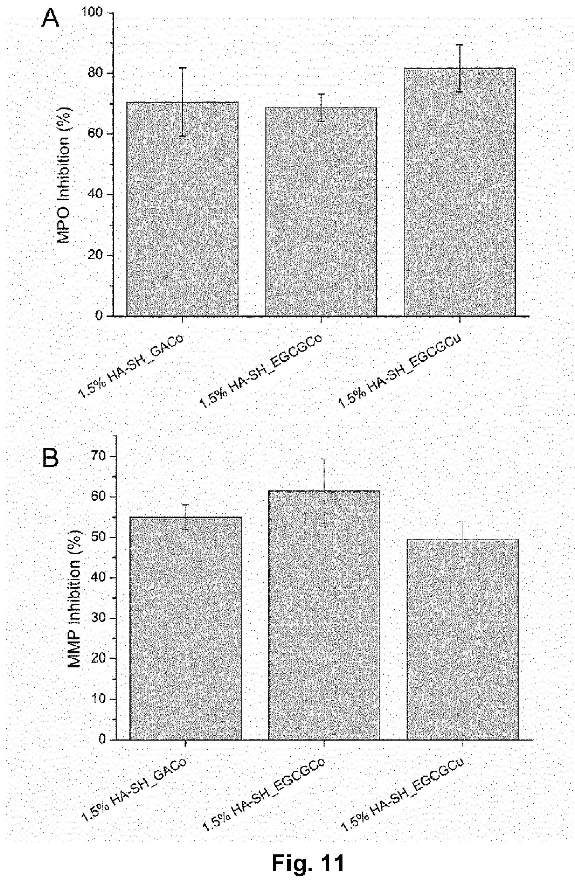

The nanocomposite hydrogels composed by HA-SH and AgLig NPs inhibited between 60 and 100 % MPO activity as a function of the amount of AgLig NPs due to the demonstrated capacity of the polyphenols to act either as HOCl scavenger or substrates in the catalytic cycle of MPO, inhibiting its chlorination activity. The highest MPO inhibition was achieved for hydrogels prepared with varying concentration of HA-SH and the highest content of AgLig NPs. Regarding the MMPs activity, the hydrogels containing hyaluronic acid and silver lignin nanoparticles (0.2 mg/ml) inhibited up to 68 % MMP independently on the amount of the HA-SH employed (Figure 10). Other thiolated hyaluronic acid hydrogels with PSNPs containing Co or Cu were able to inhibit both enzymes by 60 to 80% in the case of the MPO, and 50 to 60 % in the case of MMPs (Figure 11).

**Experiment 10: Silver release from HA-SH/AgLig NPs hydrogels**

The stability of the nanocomposite hydrogels and the cumulative release of silver from the hydrogel samples (30 mg each) was assessed in 2 mL PBS (100 mM, pH 7.4) and PBS containing hyaluronidase (10 U/mL) for 5 days at 37 °C. At defined time intervals, 800 $\mu$L of the solutions were collected and replenished with fresh PBS solution. The total amount of silver released in the solution was quantified with inductively coupled plasma mass spectrometry (ICP-MS) calibrated by internal standard with $^{115}$In and a standard curve of [107]Ag.

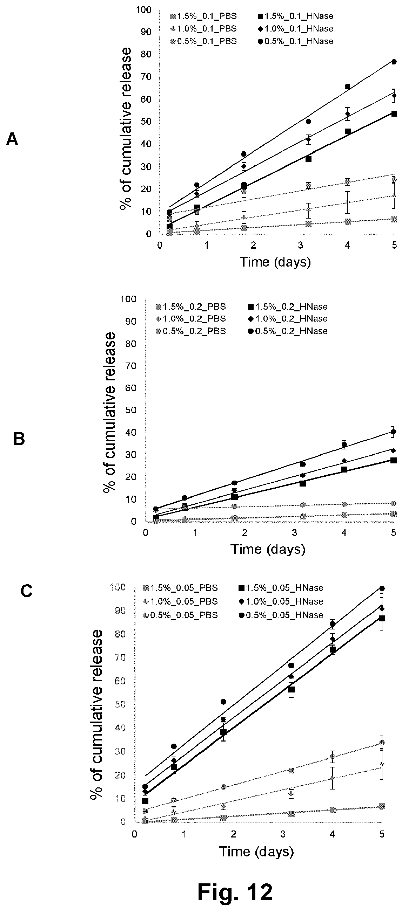

From the silver release profile (Figure 12), it was evident that the concentration of silver released from the hydrogel increased linearly in time (correlation coefficient > 0.99), indicating zero-order kinetics. The zero-order kinetics was maintained even after more than 90 % of silver was released. Initial burst release and incomplete release, which are common issues when using biodegradable hydrogels, were not observed here. Due to their more loosely crosslinked network and hence faster degradation rate, the hydrogels prepared with lower HA-SH concentration showed faster silver release, compared to those with higher HA-SH content (Figure 12). All hydrogels were stable in PBS for over 5 days of incubation (< 30 % of silver was released), being the samples prepared with lower concentration of AgLig NPs (0.05 mg/mL) and different percentage of HA-SH (0.5, 1, 1.5 %) degraded faster than the ones containing larger amount of NPs. Increasing the content of HA-SH at a fixed concentration of AgLig NPs led to enhanced stability of the hydrogels. Thus, varying the AgLig NPs to HA-SH ratio in the self-assembling reaction will allow to control the stability of the hydrogels and the release of antimicrobial active, depending on the envisaged biomedical application.

HNase is usually overexpressed due to bacterial infections. Thus, HA-based hydrogels could act as enzyme-responsive bioactive platforms to release the therapeutic cargo upon infection. Significantly, higher silver concentration and faster silver release were detected for the hydrogels incubated with HNase, compared to those in only PBS. Although the release rate changed, the zero-order kinetics remained unaltered, indicating that silver was released due to HNase digestion of the hydrogels, rather than due to diffusion through the biopolymer matrix. The different slopes in the graphs (Figure 11) in both presence or absence of HNase indicate that the silver release behavior significantly depends on the AgLig NPs content, being the hydrogel prepared with the highest HA-SH and AgLig NPs content (1.5%_0.2 HA-SH/AgLig NPs) the most stable one. In this case, the higher density of the hydrogel structure, caused by non-covalent cross-linking of AgLig NPs and HA-SH, hindered the release of silver. The zero-order kinetics and slow release will not only extend the durability of the antimicrobial effect, but will also maintain constant silver concentration maximizing the therapeutic and minimizing the toxic effects.

**Experiment 11: Biocompatibility assessment**

Keratinocytes (HaCaT cell line) were used to assess the biocompatibility of the hydrogels. The cells were maintained in Dulbecco's Modified Eagle's Medium (DMEM, Sigma-Aldrich) supplemented with 200 mM of L-glutamine, 1 % penicillin and 10 % (v/v) fetal bovine serum, at 37 °C in a humidified atmosphere with 5 % $CO_2$. At pre-confluence, the cells were harvested using trypsin-EDTA (ATCC-30-2101, 0.25 % (w/v) trypsin/0.53 mM EDTA solution in Hank's BSS without calcium or magnesium) and seeded at a density of 6 x $10^4$ cells/well on a 96-well tissue culture treated polystyrene plate (Nunc). After 24 h, the cells were washed twice with sterile PBS and incubated with UV-sterilized hydrogel samples (30 mg) for 1, 3 and 7 days at 37 °C. At the end of these periods, the samples were removed, the growth media withdrawn and the cells were washed twice with PBS and stained with 100 μL 10 % (v/v) AlamarBlue™ Cell Viability Reagent in DMEM for 4 h at 37 °C. Cells without hydrogel served as a negative control (100 % cell viability). The absorbance at 570 nm was measured with a microplate reader, setting 600 nm as a reference wavelength. All results are reported as mean values ± standard deviations (n = 3).

The cells' viability was also assessed by LIVE/DEAD® Viability/Cytotoxicity assay kit for mammalian cells. Briefly, the cells were stained with a mixture of calcein:ethidium homodimer in a ratio 4:1, prepared in PBS, for 15 min in the dark. After that, the non-reacted stains were washed with PBS, and the cells were observed using a fluorescence microscope (Nikon/Eclipse Ti-S, the Netherlands) at $\lambda_{exc}/\lambda_{em}$=494/517 nm for calcein and at $\lambda_{exc}/\lambda_{em}$=517/617 nm for ethidium homodimer-1.

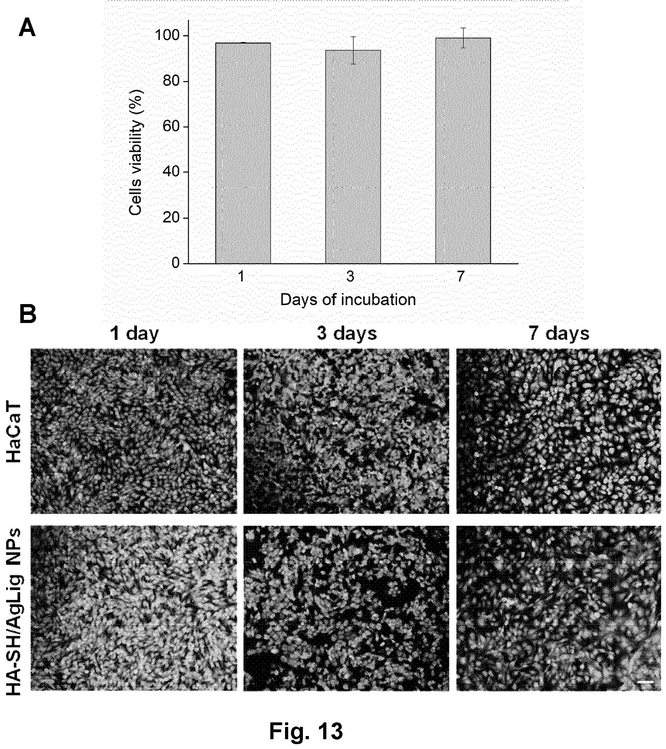

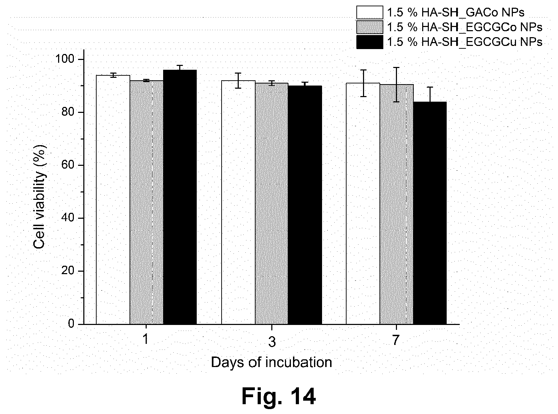

This experiment confirmed the biocompatibility of the AgLig NPs containing nanocomposite. The viability of the keratinocytes was higher than 95 % after 1, 3 and 7 days of contact with the hydrogel (Figure 13). In the case of the hydrogels with the PSNPs with copper and cobalt, the keratinocytes viability after 1, 3 and 7 days was higher than 80 % for each hydrogel demonstrating the low toxicity of the materials (Figure 14).

**Experiment 12: *In vivo* wound healing of HA-SH/AgLig NPs hydrogels**

Mouse model was used for *in vivo* validation of the wound healing potential of HA-SH/AgLig NPs hydrogel. Twelve male albino mice at the age of 3-4 months and body weight about 30 g were randomly divided into three groups of four animals. The 1st group consisted of non-diabetic mice treated with Dermazin (Sandoz) (10 mg/g) - a commercial benchmark product based on silver sulfadiazine used for prevention and treatment of wound infections, the 2nd group of non-diabetic mice treated with HA-SH/AgLig NPs hydrogel, and the 3rd group of diabetic mice treated with HA-SH/AgLig NPs hydrogel. The animals were used according to the European and national regulations for animal protection and welfare.

After sterilization with ethanol and braunol solution (7.5 g/100 g) (B. Braun, Germany), the mice were shaved and full skin thickness wounds were surgically created on the dorsal area using a sterile biopsy punch (2 mm). The surgical procedure was carried out under general anesthesia with a mixture of tiletamine/zolazepam, xylazine and butorphanol in doses of 5, 4 and 0.15 mg/kg, respectively. Before the wound creation, the mice from group 3 were injected with streptozotocin (50 mg/kg in 0.1 mL citrate buffer, pH 4.5). After 15 min, a single dose of nicotinamide (120 mg/kg in 0.1 mL saline solution) was applied to induce type 2 diabetes.As a result, the concentration of glucose in the blood of the animals was above 200 mg/dL, while the hemoglobin and hematocrit levels decreased significantly, confirming the induction of type 2 diabetes.

During the first day after the surgery, the wounds were disinfected using 3 % $H_2O_2$ solution. Thereafter, the

nanocomposite hydrogel and Dermazin were applied three times per day until the complete recovery of the wounds. During the experiment, the general appearance, normal breathing and mobility, ability and willingness to eat and drink, as well as signs of pain and disruption of life-supporting processes were monitored on a daily basis. Additionally, the presence of bacteria, signs of inflammation, re-epithelialization and healing of the wounds were checked. After 15 days, the animals were sacrificed and their skin and blood were subjected to histological and hematological evaluation, respectively.

For the histopathological examination, tissue samples ($0.5 \text{ cm}^3$ in size) from the wound area were cut and fixed in 10 % neutral buffered formalin, followed by dehydration in ethanol and embedding in paraffin. Tissue sections (3 - 5 $\mu$m thick) were stained in 1 g/L of Mayer's hematoxylin solution (Merck), hematoxylin and 1 % aqueous solution of Eosin Y (Merck), and examined by a light microscope (Leica DM 5000B, Wetzlar, Germany). To evaluate the morphological changes after the treatment with hydrogels the sections were scored for: i) presence of proliferation and granulation, ii) fibrotic tissue development, iii) vascularization and remodeling, iv) erosions, v) inflammatory reactions and other pathological lesions.

Hematological analysis was performed to study the general health status of the treated groups and evaluate the causes of discomfort observed in the diabetic mice. This was important to ascertain that the metabolic disorder is the reason for the worse general conditions in the diabetic animals when compared to the non-diabetic ones. For the tests, blood samples from the mice were taken via cardiac puncture after anesthesia with 80 mg/kg ketamine (Kepro B.V., Holland) and 0.5 mg/kg diazepam (Gerot Pharmazeutika GmbH, Austria) prepared in ethylenediaminetetraacetic acid. Complete blood count and biochemical analysis were performed using Automatic Hematology Analyzer (Mindray BC - 2800 Vet) and Blood Chemistry Analyzer (MNCHIP Celercare V2).

The antimicrobial performance of the hydrogels *in vivo* was also evaluated. Seven days after the beginning of the experiment, wound exudate was taken and placed in a sterile medium. The microbiology studies were performed in a clinical microbiology laboratory following standard protocols for microbial detection in fluid samples.

The treatment efficacy of the nanocomposite hydrogels was validated *in vivo* in an established mouse model. This model does not use specific pathogen inoculum, but rather mimics the natural process by which microorganisms colonize the wound and induce infection. After sterilization with braunol solution, full thickness skin wounds were surgically created on the dorsal area of the mice (Figure 15A). After 24 h, the nanocomposite hydrogels and Dermazin cream (the positive control) were applied on the wound site and the healing process was monitored over a period of 15 days.

Rapid contraction of the wound edges followed by complete recovery of the wounds created on the animals from groups 2 (non-diabetic mice) and 3 (diabetic mice) treated with HA-SH/AgLig NPs hydrogel were observed at the end of the experiment (Figure 15A). The cutaneous wound healing occurred with minimal hemostasis and fibrin clot formation. Furthermore, the inflammation phase was with mild inflammatory response and the wound surface and margins appeared clean and dry. Retraction of the wound until visible restoration with undetectable by naked eye scar formation and complete re-epithelization in the treated groups was achieved.

The histological analysis of the wound tissue at sacrifice showed minor scab formation and lack of any inflammation in the integument in all three groups (Figure 15B). The damaged epidermis and dermis were completely regenerated, skin defects were completely restored and even epidermal structures such as follicles and sebaceous gland were developed by the remaining basal keratinocytes and the hair follicles around the wound. High granulation and epithelial proliferation in the mice from groups 2 and 3 treated with the hydrogels were observed. Pronounced regeneration of the skin appendages was achieved in mice from group 1 (treated with Dermazin) and group 2 (threated with nanocomposite hydrogel) (Figure 15B), whereas the animals with induced diabetes mellitus (group 3) differed in presenting less focal features of hair follicles and sebaceous glands (Figure 15B). This is explained by the pathology of the diabetic wounds and the difficulties to heal due to the high glucose levels as well as the microvascular and macrovascular complications. Importantly, after 15 days of treatment, capillaries were present on the wound site in all animal groups, indicating regeneration of the skin and adequate wound healing with the hydrogels. These observations confirmed that the application of HA-SH/AgLig NPs hydrogels led to rapid and efficient wound healing, even of diabetic wounds, comparable with the effect of commercially available pharmaceutical products. Moreover, cellular damage or inflammation were not observed during the experiment, demonstrating the potential of the developed nanocomposite hydrogels to be used for wound management *in vivo*.

Hematological analysis was further performed to assess the effect of the hydrogels on the health status of the animals, potential pathology development and toxicity. In general, all animals had normal blood counts without any signs for inflammation and bacteremia. The blood parameters counts of mice from groups 1 and 2 were within the reference range and the animals were considered healthy. A decrease in the hemoglobin and hematocrit was observed in the diabetic mice from group 3 (Table 1) in agreement with the histological studies. Additionally, the test revealed lack of systemic postoperative and wound related inflammation due to bacterial infection and therefore confirmed the antimicrobial efficiency of the hydrogels. The bactericidal action of the AgLig NPs was also evidenced by the absence of bacterial contamination in the wounds over the treatment period. In summary, the *in vivo* results demonstrated tolerability and lack of local irritation or signs of toxicity of the developed hydrogels.

**Table 1**: Diabetic mouse blood parameters at the 15 day of the healing process.

| Parameter | Diabetic mouse treated with HA-SH/ AgLig NPs | Standards |
|---|---|---|
| White Blood Count ($L^{-1}$) | $5.8 \times 10^9$ | 5.05 -15.76 |
| Lymphocytes ($L^{-1}$) | $3.9 \times 10^9$ | 3.20 - 11.02 |
| Monocytes ($L^{-1}$) | $0.2 \times 10^9$ | 0 - 0.43 |
| Granulocytes ($L^{-1}$) | $1.7 \times 10^9$ | 0.75 - 3.51 |
| Lymphocytes (%) | 66.8 | |
| Monocytes (%) | 4.2 | |
| Granulocytes (%) | 99.0 | |
| Red Blood Cell ($L^{-1}$) | $6.75 \times 10^{12}$ | 6.66 -10.52 |
| Hemoglobin (g $dL^{-1}$) | 9,9 | 13.9 -15.9 |
| Hematocrit (%) | 31.6 | 39-49 |
| Mean Corpuscular Volume (fL) | 46.9 | 41.2-48.4 |
| Mean Corpuscular Hemoglobin (pg) | 14.6 | 14.1 -17.1 |
| Mean Corpuscular Hemoglobin Concentration (g $L^{-1}$) | 313 | 328 - 357 |
| Red Cell Distribution Width (%) | 20.2 | 18.8-19 |
| Platelets ($L^{-1}$) | $496 \times 10^9$ | 402 - 1098 |
| Mean Platelet Volume (fL) | 5.8 | 4.3 - 8.07 |
| Platelet Distribution Width (%) | 16.1 | 13-30 |
| Plateletcrit (%) | 0.287 | 0.2 - 0.5 |

The present invention should not be taken to be limited to the embodiments herein described.

Accordingly, the scope of the invention is defined by the following claims.

**Claims**

1. A method for obtaining an *in situ* self-assembling nanocomposite hydrogel, **characterized in that** it comprises:

   a. Mixing 75%-95% by weight of an aqueous solution of biopolymers bearing nucleophilic groups with a concentration of 5 to 20 mg/mL and pH 4.5 - 8.0 with 5-25% by weight of an aqueous solution of nanoparticles with phenolic shell (PSNPs) with a concentration of 0.02 to 0.3 mg/mL at 20°C - 50 °C;
   b. Incubating the mixture obtained in the previous step at 20°C to 50°C, for a period between 0.2 to 180 min.

2. The method according to claim 1, wherein the aqueous solution of biopolymers bearing nucleophilic groups has a concentration of 8.5 to 17.5 mg/mL.

3. The method according to claims 1 or 2, wherein the aqueous solution of nanoparticles with phenolic shell has a concentration of 0.1 to 0.2 mg/mL.

4. The method according to any of claims 1 to 3, wherein the pH of the mixture is between 5.5 to 7.0.

5. The method according to any of claims 1 to 4, wherein the temperature of the incubation is between 30-45°C.

6. A nanocomposite hydrogel **characterized in that** it comprises 75%-95% by weight of biopolymers bearing nucleophilic groups and 5-25% by weight of nanoparticles with phenolic shell (PSNPs), being the percentages referred to the total weight of the nanocomposite hydrogel.

7. The nanocomposite hydrogel according to claim 6, wherein the nucleophilic groups are selected from the group consisting of alcohols, phenols, carboxylic, amines and thiols.

8. The nanocomposite hydrogel according to claims 6 or 7, wherein the biopolymer bearing nucleophilic groups is selected from the group consisting of a polypeptide, a polysaccharide, a derivative thereof and combinations thereof.

9. The nanocomposite hydrogel according to any of claims 6 to 8, wherein the phenolic shell of the PSNPs is made of compounds with phenolic groups which are natural phenolic polymers or phenolic compounds.

10. The nanocomposite hydrogel according to any of claims 6 to 9, wherein the PSNPs comprise metal core and it is made of a metal that is selected from the group consisting of Ag, Cu, Co, Zn, Fe, Se, Te, Al, Au, V, Cr, Mn, Ni, Zr, Mo, Ru, Rh, Cd, Ce, Eu, Gd, and Tb.

11. The nanocomposite hydrogel according to any of claims 6 to 9, wherein the PSNPs comprise phenolic core and it is made of compounds with phenolic groups which are natural phenolic polymers or phenolic compounds.

12. The nanocomposite hydrogel of any of claims 6 to 11 for use as a medicament.

13. The nanocomposite hydrogel of any of claims 6 to 11 for use as an anti-bacterial medicament, or anti-inflammatory medicament or in the treatment of chronic wounds.

14. The nanocomposite hydrogel for use according to claim 13, **characterised in that** the hydrogel is administered by injection when the nanocomposite hydrogel is an injectable gel or the nanocomposite hydrogel is administered topically when the hydrogel composition is an ointment.

15. The use of the nanocomposite hydrogel of any the claims 6 to 11 for the encapsulation of human cells, growth factors, cytokines, vitamins, proteins, biomolecules or a combination thereof.

Fig. 1

**Fig. 2**

**Fig. 3**

**Fig. 4**

**Fig. 5**

Fig. 6

**Fig. 7**

Fig. 8

Fig. 9

Fig. 10

Fig. 11

Fig. 12

Fig. 13

Fig. 14

**Fig. 15**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2017/319506 A1 (APPEL ERIC A [US] ET AL) 9 November 2017 (2017-11-09) * paragraphs [0077] - [0097]; claims 1-20; examples 1-9 * | 1-15 | INV. A61L26/00 A61K9/06 A61K47/36 |
| Y,D | MORENA A. GALA ET AL: "Antibacterial Polyurethane Foams with Incorporated Lignin-Capped Silver Nanoparticles for Chronic Wound Treatment", INDUSTRIAL & ENGINEERING CHEMISTRY RESEARCH, [Online] vol. 59, no. 10, 11 March 2020 (2020-03-11), pages 4504-4514, XP055810757, ISSN: 0888-5885, DOI: 10.1021/acs.iecr.9b06362 Retrieved from the Internet: URL:https://pubs.acs.org/doi/pdf/10.1021/acs.iecr.9b06362> [retrieved on 2021-06-08] * the whole document * | 1-15 | |
| A | US 10 772 844 B2 (UNIV NAT TSING HUA [TW]) 15 September 2020 (2020-09-15) * claims 1-15; examples 1-5 * | 1-15 | TECHNICAL FIELDS SEARCHED (IPC) A61L A61K |
| A | WO 2017/149378 A1 (FUND HOSPITAL UNIV VALL D'HEBRON-INSTITUT DE RECERCA [ES] ET AL.) 8 September 2017 (2017-09-08) * claims 1-19 * | 1-15 | |

The present search report has been drawn up for all claims

| Place of search | Date of completion of the search | Examiner |
|---|---|---|
| The Hague | 9 June 2021 | Burtan, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 38 3150

This annex lists the patent family members relating to the patent documents cited in the above-mentioned European search report.
The members are as contained in the European Patent Office EDP file on
The European Patent Office is in no way liable for these particulars which are merely given for the purpose of information.

09-06-2021

| Patent document cited in search report | Publication date | Patent family member(s) | Publication date |
|---|---|---|---|
| US 2017319506 A1 | 09-11-2017 | US 2017319506 A1<br>WO 2016049360 A1 | 09-11-2017<br>31-03-2016 |
| US 10772844 B2 | 15-09-2020 | TW 202011939 A<br>US 2020093752 A1 | 01-04-2020<br>26-03-2020 |
| WO 2017149378 A1 | 08-09-2017 | AU 2017225481 A1<br>BR 112018067485 A2<br>CA 3016012 A1<br>EP 3423098 A1<br>ES 2808996 T3<br>JP 2019512479 A<br>US 2019054172 A1<br>WO 2017149378 A1 | 13-09-2018<br>12-02-2019<br>08-09-2017<br>09-01-2019<br>02-03-2021<br>16-05-2019<br>21-02-2019<br>08-09-2017 |

EPO FORM P0459

For more details about this annex : see Official Journal of the European Patent Office, No. 12/82

## REFERENCES CITED IN THE DESCRIPTION

**Patent documents cited in the description**

- CN 110835382 A
- WO 2011028031 A2
- RO 134197 A2
- CN 104255792 A
- US 10034958 B2

**Non-patent literature cited in the description**

- **A. GALA MORENA ; IVAYLO STEFANOV ; KRISTINA IVANOVA ; SÍLVIA PÉREZ-RAFAEL ; MIGUEL SÁNCHEZ-SOTO ; TZANKO TZANOV.** Antibacterial polyurethane foams with incorporated lignin-capped silver nanoparticles for chronic wound treatment. *Ind. Eng. Chem. Res.,* 2020, vol. 59 (10), 4504-4514, <https://dx.doi.org/10.1021/acs.iecr.9b06362>

- **DONGLIN GAN ; WENSI XING ; LILI JIANG ; JU FANG ; CANCAN ZHAO ; FUZENG REN ; LIMING FANG ; KEFENG WANG ; XIONG LU.** Plant-inspired adhesive and tough hydrogel based on Ag-Lignin nanoparticles-triggered dynamic redox catechol chemistry. *Nature Communications,* 2019, vol. 10 (1487, <https://doi.org/10.1038/s41467-019-09351-2>

- **SEI KWANG HAHN ; JUNG KYU PARK ; TAKASHI TOMIMATSU ; TSUYOSHI SHIMOBOJI.** Synthesis and degradation test of hyaluronic acid hydrogels. *International Journal of Biological Macromolecules,* March 2007, vol. 40 (4, 10), 374-380, <https://doi.org/10.1016/j.ijbiomac.2006.09.019>

- **HOYO J ; IVANOVA K ; TORRENT-BURGUES J ; TZANOV T.** Interaction of Silver-Lignin Nanoparticles With Mammalian Mimetic Membranes. *Front Bioeng Biotechnol.,* 2020, vol. 8, 439, <https://doi.org/10.3389/fbioe.2020.00439>

- **LI, KE et al.** Self-Assembled Metal-Phenolic Nanoparticles for Enhanced Synergistic Combination Therapy against Colon Cancer. *Advanced Biosystems.,* November 2018, vol. 3 (2), 1800241, <https://doi.org/10.1002/adbi.201800241>